# EUROPEAN PATENT SPECIFICATION

(11) **EP 4 271 360 B1**
(45) Date of publication and mention of the grant of the patent: **29.04.2026**
(21) Application number: 21848196.8
(22) Date of filing: 30.12.2021
(51) Int. Cl.: A61K 9/00, A61K 9/10, A61K 31/734, A61K 33/10

(54) **NEW COMBINATIONS AND COMPOSITIONS OF SUCRALFATE IN ALGINATE AND THEIR USE IN THERAPY**
NEUE KOMBINATIONEN UND ZUSAMMENSETZUNGEN AUS SUCRALFAT IN ALGINAT UND DEREN VERWENDUNG IN DER THERAPIE
NOUVELLES COMBINAISONS ET COMPOSITIONS DE SUCRALFATE DANS DE L'ALGINATE ET LEUR UTILISATION EN THÉRAPIE

(30) Priority: 04.01.2021 IT 202100000053
(43) Date of publication of application: 08.11.2023
(73) Proprietor: NYUMA PHARMA SRL, 28041 Arona (NO) (IT)
(72) Inventor: COLOMBO, Paolo, 28041 Arona (NO) (IT); SCARPIGNATO, Carmelo, 28041 Arona (NO) (IT); ROSSI, Alessandra, 28041 Arona (NO) (IT)
(74) Representative: Trupiano, Federica
(86) International application number: PCT/IB2021/062464
(87) International publication number: WO 2022/144825

(56) References cited:
- US-A- 5 447 918
- TYTGAT G N J ET AL: "4 The medical therapy of reflux oesophagitis", BAILLIERES CLINICAL GASTROENTEROLOGY, BAILLIERES TINDALL, LONDON, GB, vol. 1, no. 4, 1 October 1987 (1987-10-01), pages 791 - 807, XP023274080, ISSN: 0950-3528, [retrieved on 19871001], DOI: 10.1016/0950-3528(87)90019-4
- RICHARDSON J C ET AL: "Oesophageal bioadhesion of sodium alginate suspensions: particle swelling and mucosal retention", EUROPEAN JOURNAL OF PHARMACEUTICAL SCIENCES, ELSEVIER AMSTERDAM, NL, vol. 23, no. 1, 1 September 2004 (2004-09-01), pages 49 - 56, XP004795881, ISSN: 0928-0987, DOI: 10.1016/J.EJPS.2004.05.001

## Description

The present invention relates to novel pharmaceutical combinations and compositions comprising sucralfate and magnesium alginate, a process for their preparation and the use of said compositions in therapy, in particular for the treatment and/or prevention of disorders of the upper gastro-intestinal tract.

### Background art

Alginates are natural polysaccharides, often classified as dietary fibers, which are isolated from various plant species, in particular algae belonging to the *Phaeophyceae* order.

Chemically, the alginates are alginic acid salts with alkali or alkaline earth metals, particularly sodium, calcium and magnesium. They comprise D-mannuronic acid and L-guluronic acid units interconnected through 1:4 glycosidic bonds and have both homopolymeric mannuronic acid (M-blocks) or guluronic acid (G-blocks) sequences and mixed sequences. The ratio of M to G blocks is variable and related to the plant species from which the alginate was isolated.

The alginates are biomaterials with numerous applications in the science and biomedical engineering field, thanks, in particular, to their biocompatibility and ease of gelling. In particular, alginate gels are constituted by a three-dimensional lattice of long-chain molecules held together thanks to the formation, in acid environment, of areas of junction between the different chains. Gelling of alginate can be enhanced by the presence of bivalent cations, in particular calcium, which cross-link the polymer chains through a pattern called "*egg-box*"*.* The most critical factors characterizing the physical properties of the alginates and resulting hydrogels are their composition (i.e., the M/G ratio), type of sequences, length of the G-fragments and molecular weight of the chains.

The gastroesophageal reflux disease (GERD) affects up to 40% of the population in the Western countries. The gastroesophageal reflux (especially postprandial) is a physiological phenomenon but when excessive amounts of gastric contents (acid, slightly acid, bile mixed or not with food) reach the distal and/or proximal esophagus, they cause typical symptoms (such as heartburn and regurgitation) or atypical symptoms (extra-esophageal manifestations such as cough, hoarseness, laryngitis, dental erosions), which may or may not (erosive or non-erosive forms) be accompanied by lesions of the esophageal mucosa (reflux esophagitis) and - untreated - give rise to stenosis, Barrett's esophagus and esophageal adenocarcinoma.

The alginate-based formulations can offer a rapid relief of GERD symptoms and therefore are considered an appropriate option for the symptomatic treatment in cases of mild to moderate GERD. In particular, the local mechanical action of the alginate-based formulations containing carbonate and bicarbonate is based on the formation of a compact biodegradable raft of polymeric material, called a "raft", capable of floating above the gastric contents. In fact, its anti-reflux action consists in physically opposing the regurgitation towards the esophagus of the gastric content, building a semi-solid barrier resistant to the expulsion of acid material from the stomach towards the esophagus. Therefore the alginates, by acting mechanically, are able to counteract the reflux, acid or non-acid events, and can be used in patients with GERD to mechanically limit the postprandial reflux.

US5447918 describes a composition containing, among others,
- magnesium alginate
- a gel of aluminum hydroxide and magnesium carbonate
- sucralfate
- simethicone
- potassium carbonate.

In the document the sucralfate is defined as stated in the Merck Index, which gives the definition of "amorphous white powder" and for what concerns the magnesium alginate, no data is provided regarding the ratios of D-mannuronic acid to L-guluronic acid (M/G ratio). Furthermore, although the preparation technique of the preparations containing alginic acid salts is critical for the stability of the suspension and for the formation of the raft, no description is made about the way of preparation of the suspension which is crucial to achieve a liquid suspension stable to sedimentation and able to form a soft and voluminous raft.

US6395307 describes a liquid formulation based on sodium alginate and alkali metal carbonates for use in cases of reflux. In particular, the inventors focus on the possibility of developing a concentrated liquid formulation which allows a smaller volume of the dose to be taken and, at the same time, still has advantageous physical characteristics (i.e., which remains fluid enough to be poured as a liquid). In particular, US6395307 describes a composition containing high concentrations of sodium alginate, from 8 to 20%, and a carbonate of an alkali metal, wherein said sodium alginate has an M/G ratio preferably between 0.75 and 1.1. Alternatively, the composition comprises a mixture of two alginates having different ratios, from 0.9 to 1.2 and from 0.35 to 0.5. Peter W Dettmar in Drug Development and Industrial Pharmacy, August 2017, teaches that it takes three chemical reactions to make a raft: the release of alginic acid, the development of CO₂ from a carbonate and the intervention of calcium to form a strong, compact raft.

EP3184115 describes an alginate-based composition for the treatment of the gastroesophageal reflux which includes, in addition to sodium alginate and alkali or alkaline earth metal carbonates, also a tamarind extract and a bioadhesive polymer such as Carbomer, in the attempt to improve the adhesiveness to the gastrointestinal mucosa.

In the past, another product was also proposed for the treatment of the gastro-esophageal disorders providing for the use of the sucralfate, a drug constituted by a complex of sucrose octasulfate and aluminum hydroxide, which has anti-ulcer action thanks to its cytoprotective and inhibitory activity against the pepsin. Currently, the use of this compound as an anti-ulcer has been overcome by other pharmacological classes, currently on the market, such as for example the proton pump inhibitors, which effectively reduce the gastric acid secretion and which - being administrable only 1-2 times a day - have good compliance.

In fact, the neutralizing activity of the amorphous powder sucralfate against the gastric acid is modest. It is not capable of providing a rapid relief of the gastro-esophageal heartburn nor of maintaining the intra-gastric pH at adequate values to achieve a lasting symptomatic effect. On the other hand the sucralfate powder, in contact with acids, develops a remarkable bioadhesive property. In fact, once the sucralfate powder comes into contact with the ulcerative lesions caused by the acid, it transforms into an adhesive paste which can therefore carry out its own site- and cytoprotective activity locally. This activity is more easily exerted at the level of the gastric ulcers rather than at the esophageal level, because the esophageal transit of the sucralfate powder suspension is extremely rapid, especially in the orthostatic position, due to gravity.

Therefore, the use of the sucralfate powder as a mucosal protector and as an agent promoting an anti-inflammatory activity in the treatment of the reflux symptoms, has two main problems. The first problem is the poor bioadhesion of the product to the mucosa of the esophageal tract, an environment in which the acid is not physiologically present and, when present, is present in limited quantities, and the second problem is the rapid esophageal transit. In fact, as mentioned, sucralfate in the form of amorphous solid is poorly bioadhesive in the absence of acid and the transit time of the liquid or solid pharmaceutical compositions in the esophagus is from 10 to 16 seconds.

Despite being a widely studied field, there is still a need to develop a pharmaceutical composition capable of treating the symptoms and consequences of the gastroesophageal reflux in view of a more comprehensive pharmacological approach. Therefore, it would be useful to have a pharmaceutical composition capable, at the same time, not only of trying to mechanically prevent the gastric contents from passing from the stomach to the esophagus but also of exerting, after the formation of the raft by contact with the gastric acid, an antacid, anti-corrosive (i.e., cytoprotective) and anti-inflammatory action at the level of the esophageal mucosa.

### Objects of the invention

A first object of the invention is to provide novel combinations of magnesium alginate in combination with wet gel sucralfate and with a salt of the carbonic acid, and their use in the preparation of pharmaceutical compositions useful in the treatment of the gastro-esophageal mucosa.

Another object of the invention is to provide novel pharmaceutical compositions comprising magnesium alginate in combination with wet gel sucralfate and with a carbonic acid salt, which in contact with the gastric acid provide soft and voluminous rafts able to completely incorporate the sucralfate.

Another object of the invention is to provide novel compositions for use in therapy, in particular for the treatment and/or prevention of reflux disorders of the upper gastrointestinal tract, by performing a triple action as an antacid, cytoprotective and anti-inflammatory towards the gastro-esophageal mucosa.

A further object of the invention is to provide a process for the preparation of the aforementioned compositions, particularly suitable to ensure the stability to sedimentation of the suspension during the shelf-life and to form a voluminous and soft raft in contact with the gastric acid.

### Brief description of the Figures

Figures 1 and 2 show the raft-forming behavior of two representative compositions of the invention (Examples 1 and 2, respectively) after being poured into a simulated gastric acid solution (according to the U.S. Pharmacopoeia) at 37°C.
Figure 3 shows the raft-forming behavior of a commercialized composition comprising sodium alginate, sodium bicarbonate and calcium carbonate ("Gaviscon^{®} bruciore e indigestione", see Example 7) after being poured into the simulated gastric fluid solution used in the experiments of Figures 1 and 2.

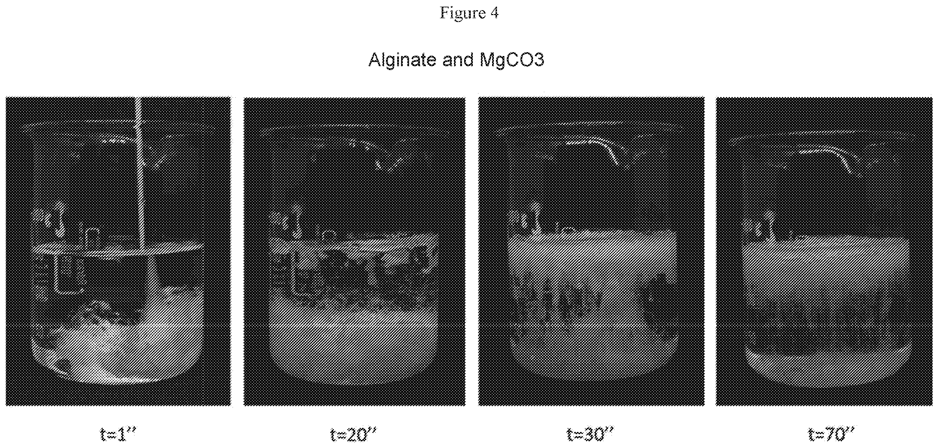

Figure 4 shows the raft-forming behavior of a composition comprising only sodium alginate and magnesium carbonate after being poured into the simulated gastric fluid solution used in the experiments of Figures 1 to 3.

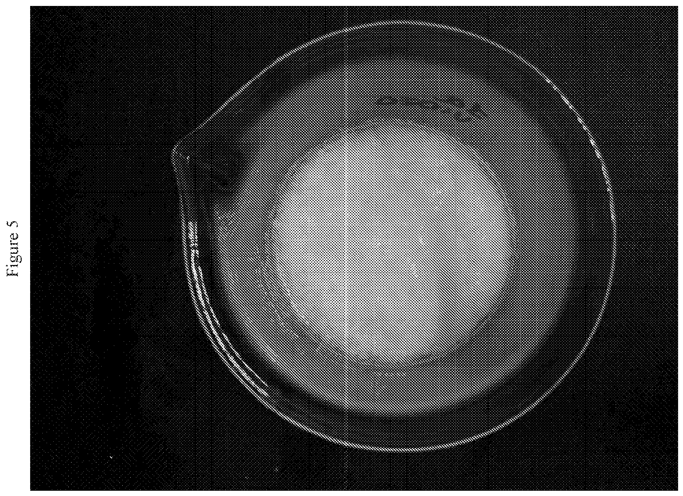

Figure 5 is a top view of the fourth frame of Figure 1.

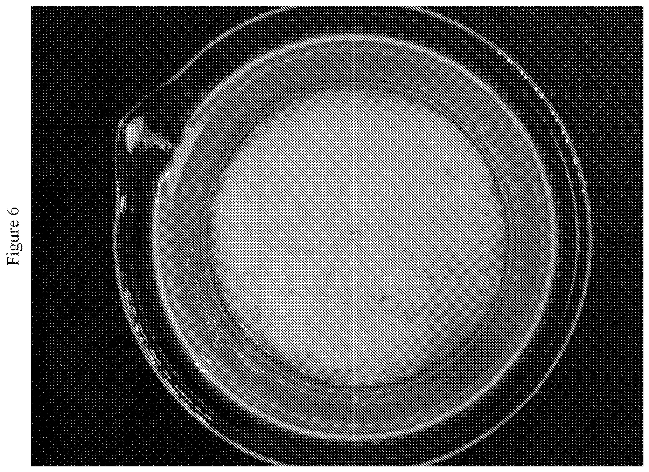

Figure 6 is a top view of the fourth frame of Figure 2.

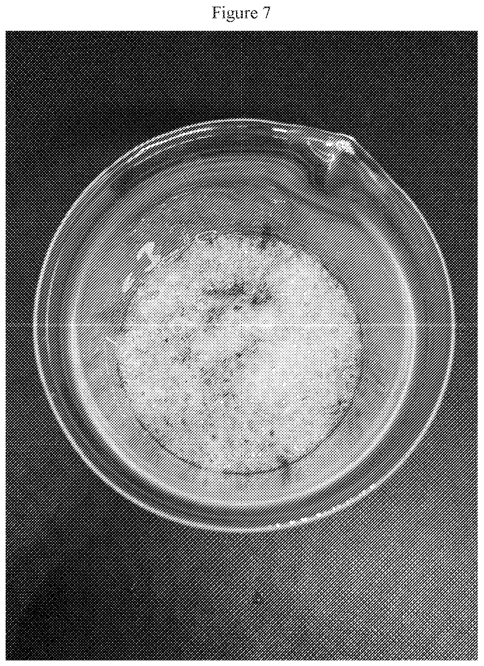

Figure 7 is a top view of the fourth frame of Figure 3.
Figure 8 shows the top and side view of the raft produced from a composition comprising sodium alginate having an M/G ratio of 0.8, sucralfate and basic magnesium carbonate, after being poured into the simulated gastric fluid solution used in the experiments of Figures 1 to 3.
Figure 9 shows the top and side view of the raft produced from a composition comprising sodium alginate having an M/G ratio of 0.8, sucralfate and basic magnesium carbonate and calcium carbonate, after being poured into the simulated gastric fluid solution used in the experiments of Figures 1 to 3.
Figure 10 shows the side view of the raft produced from a composition comprising sodium alginate having an M/G ratio of 0.6, sucralfate and basic magnesium carbonate, after being poured into the simulated gastric fluid used in the experiments of Figures 1 to 3.
Figure 11 shows the top and side view of the raft produced from a composition comprising sodium alginate having an M/G ratio of 1.5, sucralfate and basic magnesium carbonate (above) and of the same composition in which part of basic magnesium carbonate has been replaced with calcium carbonate (below).
Figure 12 shows the raft-forming behavior of the composition of the invention according to Example 5 (M/G 2.5) after being poured into the simulated gastric fluid solution used in the experiments of Figures 1 to 3.

In the Figures, "t" denotes the time in seconds (").

### Description of the invention

Subject-matter of the present invention, is a pharmaceutical combination consisting of magnesium alginate having an M/G ratio greater than 1.0, preferably from 1.5 to 3.0, wet gel sucralfate and basic magnesium carbonate.

According to a preferred embodiment, in the combination of the invention the weight ratios of magnesium alginate/sucralfate (by dry weight)/basic magnesium carbonate are 1/0.5-1/0.2-0.5, preferably about 1/0.5/0.325.

In the combination of the invention, part of the basic magnesium carbonate may be replaced with a carbonate salt of an alkali metal.

Subject-matter of the present invention is an oral pharmaceutical composition comprising the combination of the invention as defined above, together with pharmaceutically acceptable carriers and/or excipients.

Subject-matter of the present invention is an aqueous oral pharmaceutical composition comprising magnesium alginate, having an M/G ratio greater than 1.0, preferably from 1.5 to 3.0, wet gel sucralfate, basic magnesium carbonate and possibly a carbonate salt of an alkali metal.

The composition of the invention is preferably a colloidal aqueous composition.

The composition of the invention is preferably a stable colloidal aqueous composition. The composition of the invention is preferably slightly viscous, as defined below.

According to a preferred embodiment, the present invention relates to a colloidal, aqueous oral pharmaceutical composition comprising magnesium alginate having an M/G ratio greater than 1.0, preferably greater than 1.5, for example from 1.5 to 3.0, for example from 1.8 to 2.5, wet gel sucralfate, basic magnesium carbonate and possibly a carbonate salt with an alkali metal, together with one or more pharmaceutically acceptable excipients.

The colloidal, aqueous pharmaceutical composition of the invention is also referred to herein simply as the "composition".

The expression "colloidal aqueous composition" is well known in the art and refers to a stable suspension of substances in the form of particles of submicron size, mixed together in water but chemically separated. More specifically, to make a colloidal dispersion, the sucralfate product used in the colloidal composition of the invention is the wet gel sucralfate which allows making a fine colloidal dispersion of sucralfate particles in said composition, since it has been found that a great portion of the size distribution of its particles can be attributed to the colloidal dimensions, i.e. it is less than 1 micron in size.

The term "magnesium alginate" herein means the magnesium salt of the alginic acid. According to a preferred embodiment of the invention, the magnesium alginate is characterized by a ratio of mannuronic acid residues to guluronic acid residues greater 10 1.0 preferably from 1.5 to 3, for example from 1.8 to 2.8, such as 1.8: 2.0; 2.1; 2.2; 2.3; 2.4; or 2.5.

According to a particularly preferred embodiment of the invention, the magnesium alginate is characterized by an M/G ratio of mannuronic acid residues to guluronic acid residues of 1.5 or 2.5.

Even where not expressly stated, in the present description the magnesium alginate of the present invention always has M/G ratios in the ranges above.

According to a preferred embodiment of the invention, the magnesium alginate is a medium-to-high viscosity polymer, the 7.5% solution of which in water at 25°C has a viscosity from 800 to 1500 mPa s.

"Wet gel sucralfate" (below also "sucralfate gel") herein means the aluminum salt of glucose octasulfate which is in wet gel form, described in EP 0 286 978A1. Preferably, the wet gel sucralfate of the invention has a water content from 60 to 80% weight/weight, preferably from 72 to 75%, more preferably about 74%, an aluminum content of the dry product from 17 to 21% w/w and a sucrose octasulfate content, still of the dry product, from 34 to 43% w/w. Said wet gel sucralfate is a colloidal form of the sucralfate and is a raw material which has been already subjected, in the preparation step, to the polymerization of the product which is obtained by contact with the acid. Accordingly, it has the characteristics of the mentioned sucralfate paste and has a higher antacid and anti-inflammatory activity. It has been observed that such sucralfate form has particularly useful rheological characteristics to set up physically stable suspensions, as that of the invention.

The term "basic magnesium carbonate" (also known as "magnesium carbonate hydroxide") means a mixture of magnesium carbonate and magnesium hydroxide, possibly in hydrated form. Preferably, the basic magnesium carbonate contains ≥40%, more preferably 40-45% Mg (European Pharmacopoeia).

According to a preferred embodiment, said carbonate salt of an alkali metal is sodium carbonate or potassium carbonate, more preferably sodium carbonate.

The composition is an aqueous colloidal dispersion, which means that it also comprises water, preferably purified water, such as distilled or deionized water, obtained in any way. The methods to purify the water are well known in the art.

All the components of the composition are commercially available or can be prepared according to known methods and techniques.

As will be explained in more detail below, once ingested, the composition of the invention creates a special type of raft which, by positioning itself at the surface of the gastric contents, is particularly adapted for the treatment and prevention of the reflux disorders of the gastro-esophageal tract. Thus, the composition of the invention acts not only during the esophageal passage following its intake but, in the stomach, it forms a raft which, due to its volume and consistency, can be partly returned to the esophagus with the gastric contents in case of a gastro-esophageal regurgitation.

According to a preferred embodiment, the composition comprises:
- 2.5 to 7.0% magnesium alginate, preferably 3.0 to 5.5%;
- 10.0 to 40.0 % wet gel sucralfate, preferably 20.0 to 39.0%; and
- 1.0 to 7.0% basic magnesium carbonate, preferably 2.0 to 6.0%, more preferably 3.0 to 5.0%;

said % being expressed as weight of each component with respect to the total volume of the composition,
together with water and one or more additional pharmaceutically acceptable excipients.

Preferably, the wet gel sucralfate has a water content higher than 70% weight/weight, preferably about 74% weight/weight.

When present, the carbonate salt of an alkali metal, preferably sodium or potassium carbonate, more preferably sodium carbonate, replaces 1/5 to 1/3 by weight the amount of basic magnesium carbonate of the composition.

According to a preferred embodiment, in the combination and composition of the invention the weight ratio of sucralfate/magnesium alginate is 1/1, preferably about 1/0.5 (in which the sucralfate is calculated as dry product and not as wet gel sucralfate). Pharmaceutically acceptable carriers and/or excipients may optionally be added to the composition of the invention, for example components which, by interacting with the sucralfate in its gel form, improve its capability to retain the water inside the wet composition, thereby modifying the texture/consistency of the composition. In a preferred embodiment said excipient is a short chain polyol, such as mannitol, xylitol, maltitol, erythritol or sorbitol.

According to a preferred embodiment, said excipient is sorbitol, which is advantageously present in an amount from 2 to 10% by weight, with respect to the total volume of the composition. According to a preferred embodiment of the invention, for the preparation of the composition the sorbitol is preferably added in the form of an aqueous solution, for example in a 70% w/v aqueous solution. In fact it has been observed that, during the preparation of the composition of the invention, the addition of sorbitol in an aqueous solution promotes the solubilization of the alginate in water.

According to a preferred embodiment, the composition comprises, or alternatively consists of:
- 2.5 to 7.0% magnesium alginate, preferably 3.0 to 5.5%;
- 10.0 to 40.0 % wet gel sucralfate, preferably 20.0 to 39.0%; and
- 1.0 to 7.0% basic magnesium carbonate, preferably 2.0 to 6.0%, more preferably 3.0 to 5.0%;
- 2.0 a 10.0% sorbitol, preferably 4.0 to 9.0%, preferably about 7.0%;

said % being expressed as weight of each component with respect to the total volume of the composition,
together with water and one or more pharmaceutically acceptable excipients.

As mentioned above, the hereinabove sorbitol in terms of weight of sorbitol powder is preferably added to the composition in an aqueous solution, for example in a 70% w/v aqueous solution.

According to a preferred embodiment, the composition may also comprise further optional carriers and/or excipients, such as sweeteners, flavorings, preservatives for example one or more parabens or salts thereof, sodium benzoate and the like.

According to an embodiment, the invention comprises a process for preparing the composition of the invention, comprising the following steps:
i.mixing said one or more of the excipients and the magnesium alginate in water while stirring; when dissolved, dispersing the basic magnesium carbonate (and sodium or potassium carbonate, if any) and the additional optional components;
ii.mixing the wet gel sucralfate with the product obtained in step (i), making up to volume with water and homogenizing.

According to an alternative and preferred embodiment, the composition is prepared by mixing the aforementioned components in compliance with a specific predefined sequence, which has been observed to be particularly functional for the stability of the composition, preferably by preparing an aqueous solution of magnesium alginate. The wet gel sucralfate is mixed in the resulting solution. Next, the basic magnesium carbonate is added under stirring, and when present, sodium or potassium carbonate, the preservatives and optional additional carriers and/or excipients of the invention are added; the resulting composition is made up to volume with water and finally subjected to a homogenization step.

According to a particularly preferred embodiment, the invention comprises a process for preparing the composition of the invention, comprising the following steps:
a. dissolving the magnesium alginate previously mixed with the liquid sorbitol in water;
b. after the dissolution, mixing the wet gel sucralfate with the product obtained in step (a);
c. dispersing the basic magnesium carbonate (and the carbonate of an alkali metal, if any) and the additional optional components in the product obtained in step (b), making up to volume with water and homogenizing.

According to a preferred embodiment, the final mixture is homogenized using a highpressure homogenizer.

Thus, according to a particularly preferred embodiment, the invention comprises a process for preparing the composition of the invention, comprising the following steps:
a'. mixing the sorbitol solution and the magnesium alginate until a fluid paste is made; dispersing and solubilizing under stirring the fluid product achieved in water; when completely dissolved, adding sucralfate gel under stirring for at least 15 minutes;
b'. after a total stirring period of at least 30 minutes, mixing the basic magnesium carbonate with the product made in step (a');
c'. adding the possible additional excipients to the product made in step (b') and making up to volume with water and possibly homogenizing.

Preferably, the mixture of step (a') is stirred with a turbine for a few minutes, for example 15 to 50 minutes, preferably around 35 minutes, before proceeding as described in step (b').

The mixing times may of course vary depending on the quantities of composition being prepared.

It was unexpectedly found that this preferred embodiment avoids possible sedimentation phenomena of the final composition during long storage in the final containers. Without wishing to be bound by any theory, it was observed that in step (b) and (a') an interaction occurs between sucralfate and alginate, leading to cross-linking of the polymer thus increasing the physical stability of the final composition. A detailed description of the processes of the invention is provided in the Experimental Section below.

According to a preferred aspect, the composition generally has a pH of 6.0 to 8.0.

The composition is in the form of a very fine liquid suspension which, despite the presence of wet gel sucralfate in addition to magnesium alginate, is slightly viscous with a viscosity from 100 to 300 mPa·s, measured at 25°C with a SMART SERIES rotational viscometer, Rotating Spindle R2, 100 rpm (Fungilab S.A., Barcelona, SP). The composition of the invention is a pharmaceutical composition and may be packaged as single-dose form, such as for example sachets or vials, or as multi-dose form, such as bottles and the like. The single-dose forms, for example the sachets, are preferred according to the invention.

Each single dose may contain from 5 to 20 ml, preferably from 10 to 15 ml of composition, preferably 10 ml or 15 ml, more preferably 10 ml of composition.

Each single dose may preferably comprise, or alternatively consist of:
- 0.2 to 2 g sucralfate (calculated as dry form, not in the form of wet gel sucralfate, i.e. excluding the water), preferably 0.5 to 1.0 g, more preferably 1.0 g;
- 0.2 to 2 g magnesium alginate, preferably 0.5 to 1.0 g, more preferably 0.5 g; and
- 0.2 to 0.5 g basic magnesium carbonate, a portion of which may be replaced by a carbonate of an alkali metal, preferably sodium carbonate, for example a portion from 1/5 to 1/3 by weight,
together with water and one or more additional pharmaceutically acceptable excipients.

Preferably, the composition does not comprise a carbonate of an alkali metal and comprises 0.3-0.4 g of basic magnesium carbonate.

The expression "calculated as dry form and not in the wet gel sucralfate form", denotes that said amount is the title i.e. dry sucralfate and not the amount of sucralfate in wet gel form brought into the composition.

Preferably, the composition also comprises about 1 ml of aqueous solution comprising 70% w/v sorbitol for every 10-15 ml of composition.

Each single dose consists of
- 0.2 to 2 g sucralfate (calculated as dry form, not in the form of wet gel sucralfate, i.e. excluding the water), preferably 0.5 to 1.0 g, more preferably 1.0 g;
- 0.2 to 2 g magnesium alginate, preferably 0.5 to 1.0 g, more preferably 0.5 g; and
- 0.2 to 0.5 g basic magnesium carbonate, a portion of which may be replaced by a carbonate of an alkali metal, preferably sodium carbonate, for example a portion from 1/5 to 1/3 by weight,
- 0.2 to 1.0 g sucralfate, preferably 0.4 to 0.9 g, more preferably 0.7 g,
together with water and one or more additional pharmaceutically acceptable excipients.

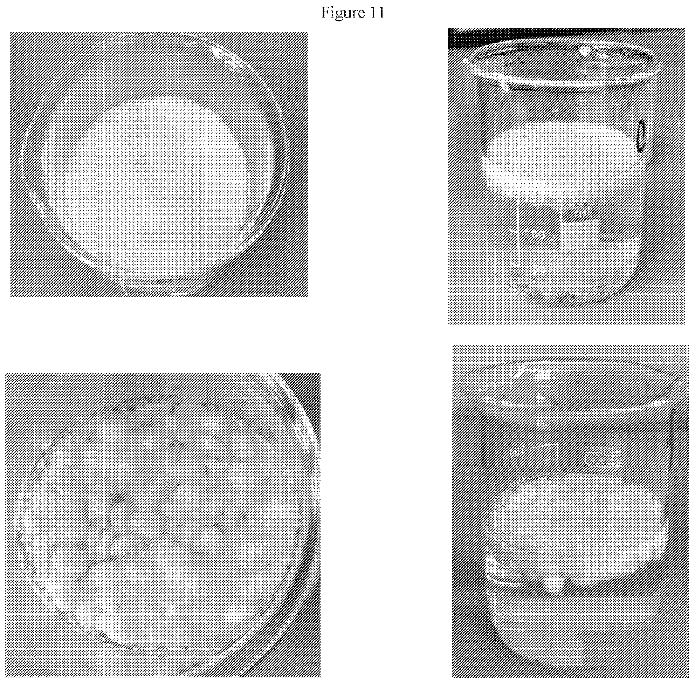

According to a preferred embodiment, the composition of the invention does not contain calcium salts, for example it does not contain calcium carbonate. As it is shown in Figure 11 and discussed below, the presence of calcium ions (calcium carbonate) does not allow the formation of the desired raft according to the invention.

According to a preferred embodiment, the composition of the invention does not contain simethicone.

According to a preferred embodiment, the composition of the invention does not contain bicarbonate salts of alkali metals.

According to another preferred embodiment, the composition of the invention is an aqueous oral composition in the form of 10 ml dosing unit, comprising, as an active ingredient, a combination of 0.5 g by dry weight of wet gel sucralfate, 0.5 g magnesium alginate and 0.217 g basic magnesium carbonate, together with pharmaceutically acceptable carriers and excipients.

According to another preferred embodiment, the composition of the invention is an aqueous oral composition in the form of 10 ml dosing unit, comprising, as an active ingredient, a combination of 1.0 g by dry weight of wet gel sucralfate, 0.5 g magnesium alginate and 0.217 g basic magnesium carbonate, together with pharmaceutically acceptable carriers and excipients.

The composition is particularly useful for treating and/or preventing the disorders of the upper gastro-intestinal tract, such as the esophageal reflux, esophagitis, gastritis, dyspepsia, peptic ulcer and the like, as it gives rise on contact with the acid to a voluminous, soft raft which completely incorporates the sucralfate gel.

The invention, according to another of the aspects thereof, refers to a composition for the use in the treatment and/or prevention of the disorders of the upper gastro-intestinal tract, such as the esophageal reflux disease (erosive and non-erosive), reflux esophagitis, eosinophilic esophagitis, mycotic esophagitis, gastritis, dyspepsia, peptic ulcer and other acid-related conditions.

The invention, according to another of the aspects thereof, refers to a composition for the use as an antacid, cytoprotective and anti-inflammatory agent for the disorders of the upper gastro-intestinal tract.

Another subject-matter of the invention is a method for treating and/or preventing the disorders of the upper gastro-intestinal tract, such as the esophageal reflux disease (erosive and non-erosive), reflux esophagitis, eosinophilic esophagitis, mycotic esophagitis, gastritis, dyspepsia, peptic ulcer and other acid-related conditions, comprising administering an effective amount of the composition to a subject in the need thereof.

The composition is intended to be administered to mammals, humans in particular, but may also be useful for pets, such as dogs and cats, and other mammals, such as livestock, for example cows, sheep, horses and the like.

For the treatment and/or prevention of the disorders of the upper gastro-intestinal tract, such as disorders due to gastro-esophageal reflux and the like, the composition may be administered several times per day, daily in between meals and before bedtime, or as needed, according to the need. This posology, which is made possible thanks to the particular colloidal and bioadhesive properties of the composition, increases the compliance of the product and is a very significant advantage of the composition over the known art.

In fact, the composition is a liquid, slightly viscous product, which by means of a mechanical action of a layer or "raft" of alginate which forms in an acid environment, characterized by a high thickness and a soft and light consistency, enhances the muco-protective and repair-promoting actions of the sucralfate gel, which are associated with the neutralization capability of the gastric acid.

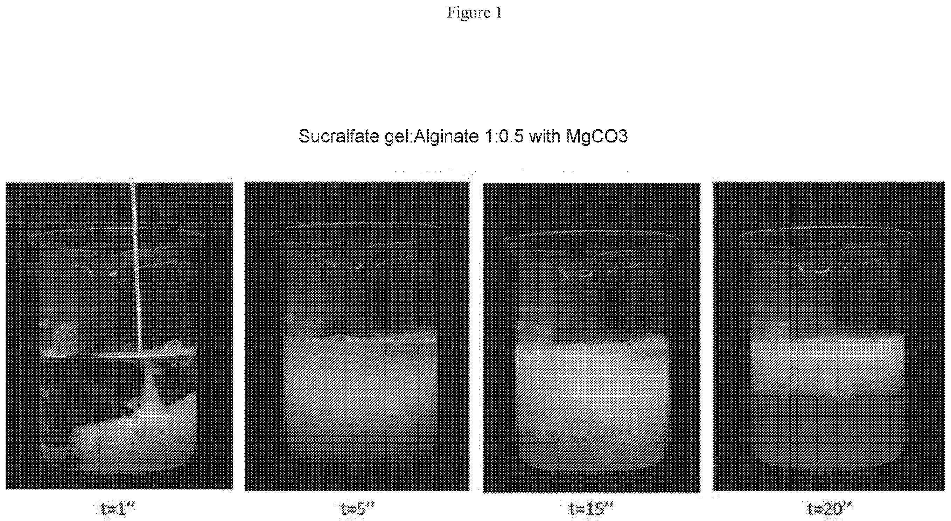

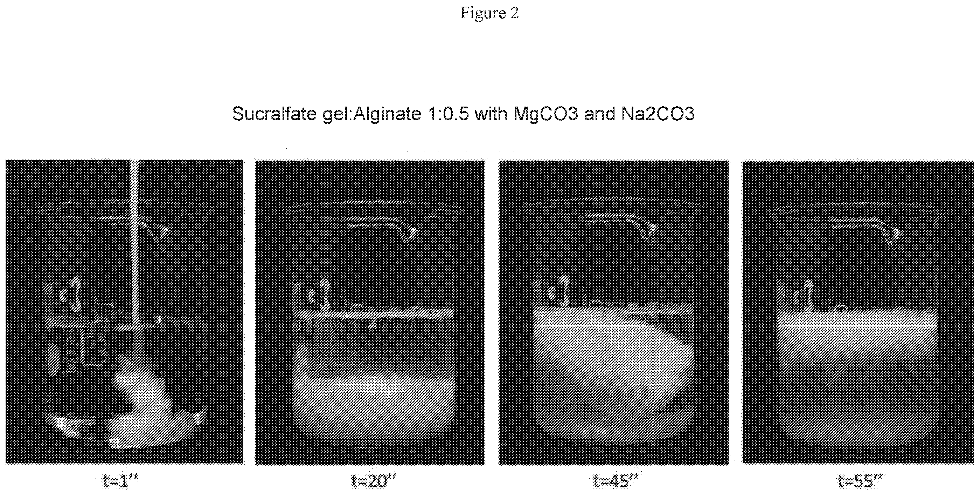

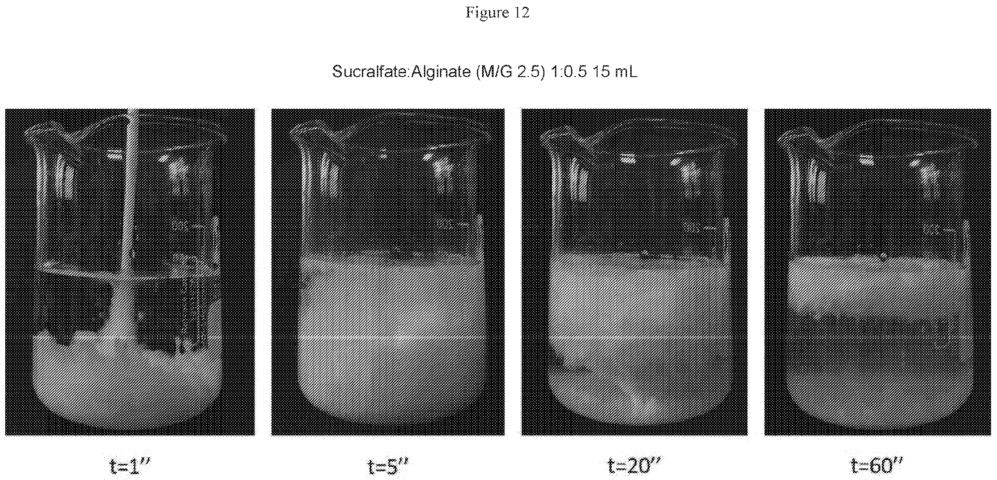

The composition, when poured into a liquid simulating the acid gastric contents, as can be seen in Figures 1, 2 and 12 attached, referring to the Examples 1, 2 and 4, surprisingly forms a thick, light and soft layer of sucralfate gel in an alginate matrix which, floating, homogeneously covers the surface of the liquid in which it was poured.

Accordingly, when administered to a subject during swallowing, the composition with sucralfate gel in the slightly viscous polymeric alginate solution crosses the esophagus, slipping over the esophageal mucosa and covering it. Once reached the stomach, the composition, by reacting with the acid that is present, gives rise to the formation of said voluminous, light and soft layer of sucralfate gel in the alginate matrix, which is floating on the fluid contained in the stomach, physically in the proximity of the esophagus-gastric junction, thus creating the conditions to prolong the adhesion of sucralfate gel to the esophagus walls. The cardial localization of the product is strategic, since during the episodes of gastro-esophageal reflux, the sucralfate gel incorporated in the layer of light and soft consistency, like a cloud, can reflux into the esophagus, thus allowing a novel contact of the bioadhesive drug with the esophageal mucosa. Therefore, the esophageal wall comes into contact with the sucralfate gel both directly and retrogradely.

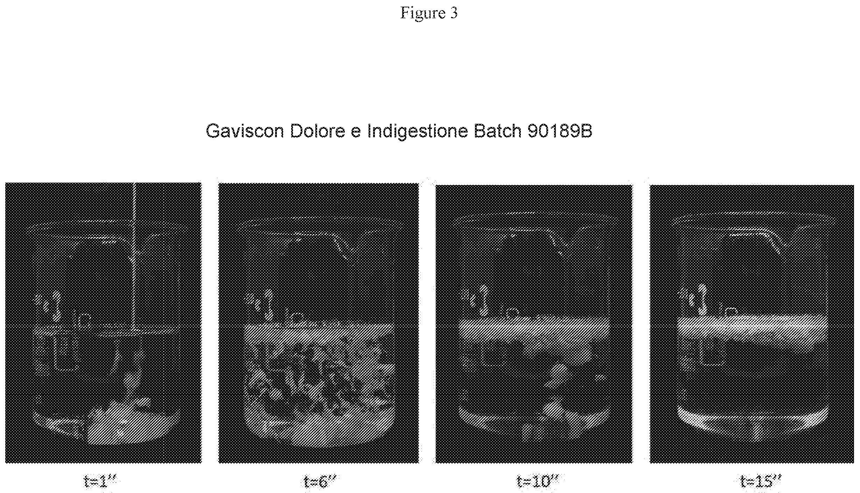

In contrast to this, as can be seen in Figure 3, the product of the prior art comprising only sodium alginate, sodium bicarbonate and calcium carbonate, under the same conditions with respect to the claimed composition, results in the formation of a layer of material floating at the surface, which is inhomogeneous in the structure, very consistent and less resilient to displacement and significantly less thick, which, although capable of floating above the gastric contents, badly lends to the possibility of flowing back into the esophagus. Furthermore, since it does not include the sucralfate gel dispersed in the alginate matrix, said product layer can only mechanically interfere with the reflux of the stomach but cannot perform the muco-protective and restorative effects, which are exerted by the presence of the sucralfate gel.

Therefore, a composition capable of providing esophageal bioadhesion of the sucralfate also in the absence of acid was identified, thanks to the physical form of wet gel of the sucralfate which has the bioadhesive capability regardless of the presence of acid. This is a way to increase the persistence of the sucralfate itself on the wall of the esophagus, where the materials of the gastric reflux arrive. Unexpectedly, this solution was identified in a low-viscous liquid formulation of wet gel sucralfate in admixture with a polymer such as the alginate. By passing the wet gel sucralfate slowly through the esophageal tract and keeping the sucralfate at a gastric location from where it can reflux into the esophagus, a new mechanism for the treatment of the pathological gastro-esophageal reflux and related disorders has been found. Thus, the composition enables the treatment and protection of the esophageal wall, not only during swallowing of the same, but also after the entrance of the composition into the stomach.

The thick light and soft layer of sucralfate gel in the alginate matrix, which forms after the administration of the composition and enables it to float on the gastric fluid, is due to the inclusion in the formulation of a carbon dioxide source that is less reactive than the bicarbonates. In fact, the effervescence has the role of providing the floating capability, which maintains the composition on the surface of the fluid contained in the stomach. The basic magnesium carbonate, even possibly in admixture with an alkali metal carbonate, proved to be extremely effective for controlled development of carbon dioxide in a strongly acid solution. The presence of a portion of alkaline metal carbonate, such as sodium or potassium carbonate, enables the formation of the carbon dioxide to be optimized.

A peculiar and surprising effect of the composition was observed with respect to the presence of aluminum which is a poly-cation that could play the same cross-linking effect as calcium to the alginate. The calcium ion causes the cross-linking of the alginate polymer chains and the formation of a resistant and compact "raft". In the claimed composition, it was unexpectedly observed that the aluminum ion present in the wet gel sucralfate determines the formation of the voluminous, homogeneous and soft floating alginate layer in which all of the sucralfate gel is kept incorporated. Such layer has a voluminous, homogeneous and soft structure, never previously described for this type of products, due to the sucralfate. In fact, it has been verified that, when the sucralfate is not present in the described composition, the polymer layer on the surface of the acid solution has an inhomogeneous, substantially slightly unstructured and more fibrous appearance which reminds of the gelatinous structure of a jellyfish. Thus, the aluminum ions present in the wet gel sucralfate are available in a useful amount to cross-link the alginate chains for the formation of a floating, cloud-light layer that holds all of the sucralfate present in the composition. The contribution of the aluminum ions is milder for the formation of this layer or raft, unlike the calcium ions which give rise to resistant and compact rafts. This result, which has never been described, was not predictable and allowed to discover a novel physical mechanism for the treatment of the gastro-esophageal reflux disease (in its various phenotypic forms) which is the possibility to reflux into the esophagus an active product such as sucralfate gel, thanks to the soft, voluminous and very light structure of the alginate layer.

Finally, it was also found that the composition, although it is a suspension of solid in a liquid that could separate during the preparation and storage, placed in a 50 ml cylinder with a cap and left to stand over a period of 4 weeks, does not have any separation by sedimentation of the solid phase of the suspension, although the viscosity is not very high. This stability is ensured when preparing the composition according to the preferred process of the invention, in particular by scrupulously following the addition sequence of its components.

Finally, the therapeutic activity of an antacid preparation depends not only on the absolute acid neutralization, but also on the rate of acid neutralization. Another novel characteristic of the composition of the invention is the achievement of an antacid activity of the sucralfate gel and alginate composition, also thanks to the presence of basic magnesium carbonate.

The antacid efficacy of the composition was measured as shown in Chapter XVI of the USP "Antacid Efficacy". In the preliminary antacid test, 15 ml of the product composition of Example 1, placed in contact with 10 ml of 0.5 N hydrochloric acid, resulted in a pH value exceeding the specific neutralization rate threshold set by the preliminary USP antacid test of 3.5.

It was also found that the composition, in addition to the neutralization rate that allows exceeding the pH value of 3.5, has an acid neutralizing capability of 19.5-20.5 milliequivalents. This value is higher than that measured under the same conditions for the commercial product of Example 3, although sucralfate is not considered an antacid agent.

In addition, the surface of the floating layer of sucralfate in alginate, which forms on a 0.1 N hydrochloric acid solution, has a pH value from 5.0 to 6.0, which is a very important value for buffering the acid in the upper part of the stomach and, consequently, in the reflux material in case of burping episodes.

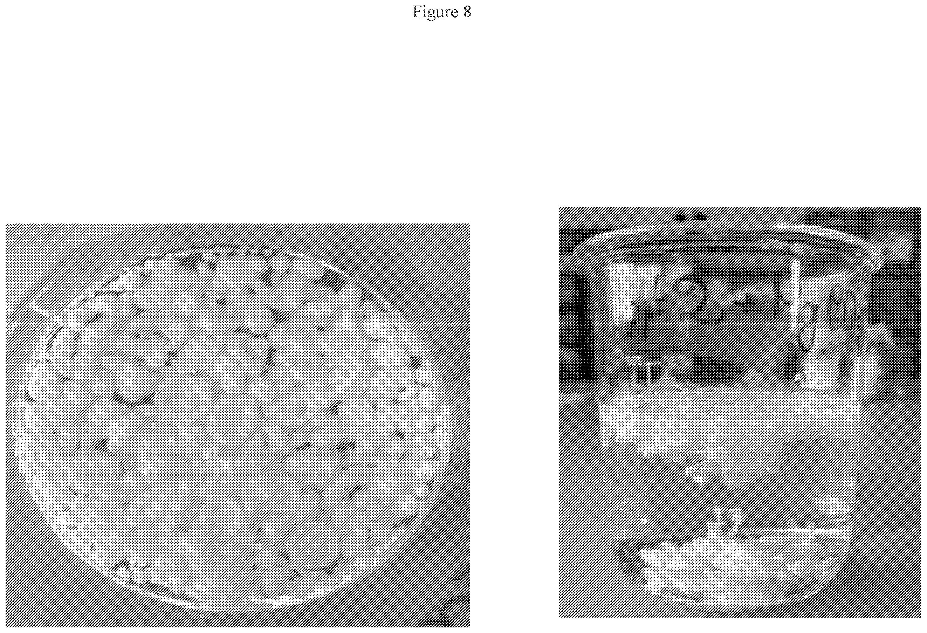

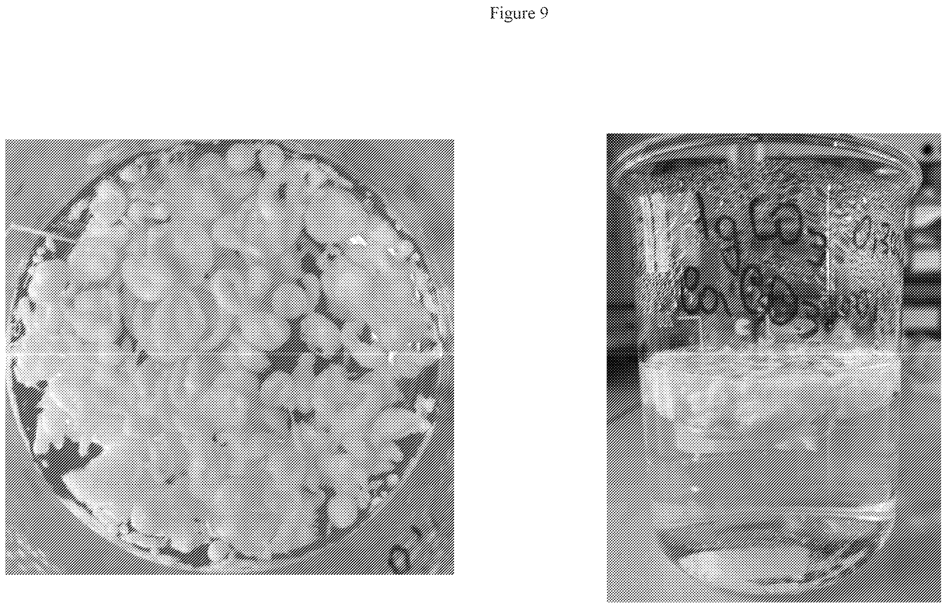

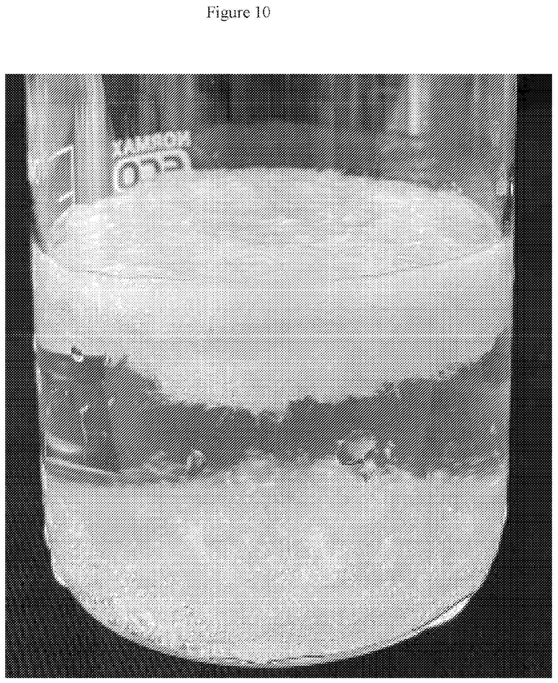

For what concerns the importance of the M/G ratio, the Figures 8 to 10 show the poor capacity of the compositions comprising magnesium alginate having a low M/G ratio (0.8; 0.8 and 0.6, respectively) to form rafts adapted to the treatment the composition is intended for. In fact, as it can be seen in Figures 8 to 10, in contact with the acid solution the formation of fragmented clots is observed, visually more consistent than the ones formed with the magnesium alginates having M/G ratio much higher than 1. With M/G ratios of about 0.8, the formation of aggregates is observed (Figures 8 and 9) whereas with the 0.6 ratio filaments are formed which remain in part at the bottom of the beaker and in part float, and the floating material is voluminous but not compact and not cross-linked.

Furthermore, the presence of basic magnesium carbonate and the absence of calcium ions, for example the calcium carbonate, in the composition of the invention is crucial. In fact, as it can be noted from Figure 11, the addition of calcium carbonate leads to a raft which is not as compact and soft as desired, which is characterized by the presence of globules cross-linked to each other. Such raft it is definitely unsuitable for the treatment the composition is intended for.

The invention will now be illustrated by the following examples, which are not intended to limit the scope of protection of the invention.

### Experimental section

In addition to the components set forth in the compositions of the Examples described below, there may be additional excipients such as for example sweeteners and/or flavorings.

In the compositions of the Examples described below, the amounts of sorbitol are shown with reference to powdered sorbitol but such component was added in 70% w/v aqueous solution.

### Example 1

A composition with a sucralfate/magnesium alginate ratio = 1/0.5 was produced, comprising:

| Component | Grams | % w/v |
|---|---|---|
| Sucralfate (of dry form) | 1.0 | 6.67 |
| as wet gel sucralfate | 3.85 | 25.6 |
| 1.5 M/G magnesium alginate | 0.5 | 3.33 |
| Sorbitol | 0.70 | 4.67 |
| Basic magnesium carbonate | 0.325 | 2.17 |
| Sodium methyl para-hydroxybenzoate | 0.04 | 0.27 |
| Sodium propyl para-hydroxybenzoate | 0.004 | 0.027 |
| Sodium benzoate | 0.03 | 0.20 |
| Water | q.s. to 15 ml | q.s. to 100 ml |

### Procedure for preparing 100 ml of the composition of the invention

### 1. Preparation of the magnesium alginate solution

In a 250 ml beaker, 4.67 g of sorbitol dissolved at 70% in water and 3.33 g of magnesium alginate were mixed to form a fluid paste. 60 ml of purified water is added while stirring with an IKA TP18/10 ULTRA-TURRAX^{®} disperser, probe diameter 13 mm, speed 1, until a homogeneous solution is formed.

### 2. Introduction of the wet gel sucralfate in the alginate solution

25.6 g of wet gel sucralfate (water content 19.0 g; dry sucralfate 6.6 g) was added to the alginate solution, in a time interval of about 15 minutes, by mixing with the ULTRA-TURRAX^{®} disperser for a total time of at least 30 minutes.

### ii. Preparation of the final composition of the invention of sucralfate and magnesium alginate

2.17 g of basic magnesium carbonate was then dispersed in the alginate/sucralfate mixture by stirring for 10 minutes. Finally, 0.27 g of sodium methyl parahydroxybenzoate, 0.027 g of sodium propyl parahydroxybenzoate and 0.2 g of sodium benzoate and purified water are added to make up the final volume of 100 ml. The final suspension was homogenized with IKA DIGITAL ULTRA-TURRAX^{®}, probe diameter 13 mm, speed 1, for 10 minutes until visually homogeneous dispersion.

### Example 2

A composition comprising sodium carbonate having a sucralfate/magnesium alginate ratio = 1/0.5 was produced:

| Component | Grams | % w/v |
|---|---|---|
| Sucralfate (of dry form) | 1.0 | 6.67 |
| as wet gel sucralfate | 3.85 | 25.6 |
| 1.5 M/G magnesium alginate | 0.5 | 3.33 |
| Sorbitol | 0.70 | 4.67 |
| Sodium carbonate | 0.108 | 0.72 |
| Basic magnesium carbonate | 0.216 | 1.44 |
| Sodium methyl para-hydroxybenzoate | 0.04 | 0.27 |
| Sodium propyl para-hydroxybenzoate | 0.004 | 0.027 |
| Sodium benzoate | 0.03 | 0.20 |
| Water | q.s. to 15 ml | q.s. to 100 ml |

### Example 3

A composition having a sucralfate/magnesium alginate ratio = 1/1 was produced, comprising:

| Component | Grams | % w/v |
|---|---|---|
| Sucralfate (of dry form) | 0.5 | 5 |
| as wet gel sucralfate | 1.92 | 19.2 |
| 1.5 M/G magnesium alginate | 0.5 | 5 |
| Sorbitol | 0.35 | 3.5 |
| Basic magnesium carbonate | 0.217 | 2.17 |
| Sodium methyl para-hydroxybenzoate | 0.03 | 0.3 |
| Sodium propyl para-hydroxybenzoate | 0.003 | 0.03 |
| Sodium benzoate | 0.02 | 0.20 |
| Water | q.s. to 10 ml | to 100 ml |

### Example 4

### Preferred process of the invention

300 ml of a suspension for the composition according to the invention has been prepared

| Component | X 300 ml |
|---|---|
| Wet gel sucralfate | 81 g |
| 2.5 M/G magnesium alginate powder | 11.475 g (residual water 14.72%) |
| Sorbitol 70% w/v aqueous solution | 20.0 ml |
| Basic magnesium carbonate (Carlo Erba) | 6.51 g |
| Sodium methyl para-hydroxybenzoate | 0.81 g |
| Sodium propyl para-hydroxybenzoate | 0.081g |
| Sodium benzoate | 0.6 g |
| Water | q.s. to 300 ml |

### 1. Preparation of the magnesium alginate solution

In a 500 ml beaker, 20 ml of sorbitol 70% w/v aqueous solution and 11.475[US1] of magnesium alginate are gradually kneaded by means of a spatula until a fluid, homogeneous compound is obtained. The compound obtained, without interposing a lot of time but gradually, is dispersed and dissolved in 100 ml of purified water. Stirring with a disperser (ULTRA-TURRAX^{®} or Silverson) is kept until a homogeneous solution of magnesium alginate is obtained.

### 2. Addition of the sucralfate gel

Separately, 81 g of sucralfate gel is weighed and added under stirring in about 15 minutes to the alginate solution. The dispersion is made with ULTRA-TURRAX^{®} or Silverson, by stirring in the first case at 7800 rpm for at least thirty minutes until homogeneousness, maintaining the temperature controlled.

### 3. Addition of the basic magnesium carbonate

Next, 6.51 g of basic magnesium carbonate is wet with about 20 ml of water until a "slurry" is formed and added to the suspension of sucralfate gel and magnesium alginate. Use Silverson or ULTRA-TURRAX^{®} to homogeneously disperse the addition of basic magnesium carbonate.

### 4. Completion of the suspension

Finally, sodium benzoate, sodium methyl parahydroxybenzoate and sodium propyl parahydroxybenzoate are dissolved in this order in the sucralfate gel/magnesium alginate/magnesium carbonate suspension, under stirring until a homogeneous dispersion is formed. Made up to a volume of 300 ml, the ULTRA-TURRAX^{®} disperser was activated for 5 minutes.

The temperature of the suspension before using ULTRA-TURRAX^{®} is checked and during homogenization at 7800 rpm it is checked that 45°C is not exceeded.

### 5. Homogenization of the suspension with PANDA

A portion of the sucralfate gel/alginate suspension, Batch Pr01-050721 was homogenized by using GEA Lab Homogenizer PandaPLUS 2000. Two cycles lasting 30 seconds were performed at a pressure of 750 bars. The final temperature of the sample was 35°C. The formulation appeared more fluid.

### Example 5

A composition having a sucralfate/magnesium alginate ratio = 1/0.5 was produced, comprising:

| Component | Grams | % w/v |
|---|---|---|
| Sucralfate (of dry form) | 1.0 | 10 |
| as wet gel sucralfate | 2.78 | 27.84 |
| 2.5 M/G magnesium alginate | 0.5 | 5 |
| Sorbitol | 0.7 | 7.0 |
| Basic magnesium carbonate | 0.325 | 3.25 |
| Sodium methyl para-hydroxybenzoate | 0.03 | 0.3 |
| Sodium propyl para-hydroxybenzoate | 0.003 | 0.03 |
| Sodium benzoate | 0.02 | 0.20 |
| Water | q.s. to 10 ml | to 100 ml |

### Example 6

A composition having a sucralfate/magnesium alginate ratio = 1/0.5 was produced, comprising:

| Component | Grams | % w/v |
|---|---|---|
| Sucralfate (of dry form) | 1.0 | 6.67 |
| as wet gel sucralfate | 2.78 | 18.57 |
| 2.5 M/G magnesium alginate | 0.5 | 3.33 |
| Sorbitol | 0.70 | 4.67 |
| Basic magnesium carbonate | 0.325 | 2.17 |
| Sodium methyl para-hydroxybenzoate | 0.04 | 0.27 |
| Sodium propyl para-hydroxybenzoate | 0.004 | 0.027 |
| Sodium benzoate | 0.03 | 0.20 |
| Water | q.s. to 15 ml | q.s. to 100 ml |

### Example 7

A composition having a sucralfate/magnesium alginate ratio = 1/1 was produced, comprising:

| Component | Grams | % w/v |
|---|---|---|
| Sucralfate (of dry form) | 0.5 | 3.33 |
| as wet gel sucralfate | 1.92 | 12.8 |
| 2.5 M/G magnesium alginate | 0.5 | 3.33 |
| Sorbitol | 0.70 | 4.67 |
| Basic magnesium carbonate | 0.325 | 2.17 |
| Sodium methyl para-hydroxybenzoate | 0.04 | 0.27 |
| Sodium propyl para-hydroxybenzoate | 0.004 | 0.027 |
| Sodium benzoate | 0.03 | 0.20 |
| Water | q.s. to 15 ml | q.s. to 100 ml |

### Example 8

In order to show the difference between compact raft and the floating layer of the claimed composition, a raft formation test was videotaped with the commercial product "Gaviscon^{®} Bruciore e Indigestione", production batch 90189B expiration 3/2021. Some frames of said video are shown in Figure 3.

## Claims

1. A combination consisting of:
- magnesium alginate having a ratio of mannuronic acid residues with respect to guluronic acid residues (M/G) greater than 1.0, preferably 1.5 to 3.0;
- wet gel sucralfate; and
- basic magnesium carbonate.

2. The combination according to claim 1, **characterized in that** the weight ratios of magnesium alginate/sucralfate by dry weight/basic magnesium carbonate are 1/0.5-1/0.2-0.5.

3. An aqueous oral pharmaceutical composition comprising the combination according to claim 1 o 2, together with one or more pharmaceutically acceptable carriers and/or excipients.

4. The composition according to claim 3, **characterized in that** it also comprises a carbonate salt of an alkali metal.

5. The composition according to claim 3 or 4, **characterized in that** said magnesium alginate has an M/G ratio greater than or equal to 1.5, preferably from 1.5 to 3.

6. The composition according to claim 5, **characterized in that** said ratio is 2.5.

7. The composition according to any one of claims 3 to 6, **characterized in that** said sucralfate is wet gel sucralfate with a water content from 60 to 80% w/w.

8. The composition according to any one of claims 3 to 7, **characterized in that** said carbonate salt of an alkali metal is sodium carbonate.

9. The composition according to any one of claims 3 to 8, **characterized in that** it comprises:
- 2.5 to 7.0% magnesium alginate, preferably 3.0 to 5.5%;
- 10.0 to 40 % wet gel sucralfate, preferably 20.0 to 39.0%; and
- 1.0 to 7.0% basic magnesium carbonate, preferably 2.0 to 6.0%, more preferably 3.0 to 5.0%; the % being expressed as weight of the component with respect to the total volume of the composition, together with water and one or more pharmaceutically active excipients.

10. The composition according to claim 8, **characterized in that** the carbonate salt of an alkali metal, when present, replaces 1/5 to 1/3 by weight of the basic amount of magnesium carbonate in the composition.

11. The composition according to any one of claims 3 to 10, **characterized in that** it is in monodose or multidose form.

12. The composition according to claim 11 **characterized in that** it is in monodose form and each individual dosing unit comprises:
- 0.2 to 2 g sucralfate, as dry form, not in the form of wet gel sucralfate, preferably 0.5 to 1.0 g;
- 0.2 to 2 g magnesium alginate, preferably about 0.5 g; and
- 0.2 to 0.5 g basic magnesium carbonate.

13. The composition according to any one of claims 3 to 12, **characterized in that** it is a colloidal aqueous composition.

14. The composition according to any one of claims 3 to 13, for the use in human and veterinary therapy.

15. The composition for the use according to claim 14, in the treatment and/or prevention of upper gastrointestinal tract disorders, esophageal reflux, esophagitis, gastritis, dyspepsia and peptic ulcer, and as an antacid, cytoprotective and anti-inflammatory agent of the gastroesophageal mucosa.

## Patentansprüche

1. Kombination, bestehend aus:
- Magnesiumalginat mit einem Verhältnis der Mannuronsäurereste zu den Guluronsäureresten (M/G) von größer als 1,0, vorzugsweise 1,5 bis 3,0;
- Sucralfat-Feuchtgel; und
- basischem Magnesiumcarbonat.

2. Kombination nach Anspruch 1, **dadurch gekennzeichnet, dass** die Gewichtsverhältnisse von Magnesiumalginat/Sucralfat nach Trockengewicht/basischem Magnesiumcarbonat 1/0,5-1/0,2-0,5 betragen.

3. Wässrige orale pharmazeutische Zusammensetzung, umfassend die Kombination nach Anspruch 1 oder 2 zusammen mit einem oder mehreren pharmazeutisch verträglichen Trägern und/oder Hilfsstoffen.

4. Zusammensetzung nach Anspruch 3, **dadurch gekennzeichnet, dass** sie ferner ein Carbonatsalz eines Alkalimetalls umfasst.

5. Zusammensetzung nach Anspruch 3 oder 4, **dadurch gekennzeichnet, dass** das Magnesiumalginat ein M/G-Verhältnis von größer oder gleich 1,5, vorzugsweise von 1,5 bis 3, aufweist.

6. Zusammensetzung nach Anspruch 5, **dadurch gekennzeichnet, dass** das Verhältnis 2,5 beträgt.

7. Zusammensetzung nach einem der Ansprüche 3 bis 6, **dadurch gekennzeichnet, dass** das Sucralfat ein Sucralfat-Feuchtgel mit einem Wassergehalt von 60 bis 80 Gew.-% ist.

8. Zusammensetzung nach einem der Ansprüche 3 bis 7, **dadurch gekennzeichnet, dass** das Carbonatsalz eines Alkalimetalls Natriumcarbonat ist.

9. Zusammensetzung nach einem der Ansprüche 3 bis 8, **dadurch gekennzeichnet, dass** sie umfasst:
- 2,5 bis 7,0 % Magnesiumalginat, vorzugsweise 3,0 bis 5,5 %;
- 10,0 bis 40 % Sucralfat-Feuchtgel, vorzugsweise 20,0 bis 39,0 %; und
- 1,0 bis 7,0 % basisches Magnesiumcarbonat, vorzugsweise 2,0 bis 6,0 %, mehr bevorzugt 3,0 bis 5,0 %;
wobei die % als Gewicht der Komponente bezogen auf das Gesamtvolumen der Zusammensetzung ausgedrückt sind, zusammen mit Wasser und einem oder mehreren pharmazeutisch aktiven Hilfsstoffen.

10. Zusammensetzung nach Anspruch 8, **dadurch gekennzeichnet, dass** das Carbonatsalz eines Alkalimetalls, sofern vorhanden, 1/5 bis 1/3 nach Gewicht der basischen Menge an Magnesiumcarbonat in der Zusammensetzung ersetzt.

11. Zusammensetzung nach einem der Ansprüche 3 bis 10, **dadurch gekennzeichnet, dass** sie in Einzeldosis- oder Mehrdosisform vorliegt.

12. Zusammensetzung nach Anspruch 11, **dadurch gekennzeichnet, dass** sie in Einzeldosisform vorliegt und jede einzelne Dosiereinheit umfasst:
- 0,2 bis 2 g Sucralfat in trockener Form, nicht in Form von Sucralfat-Feuchtgel, vorzugsweise 0,5 bis 1,0 g;
- 0,2 bis 2 g Magnesiumalginat, vorzugsweise etwa 0,5 g; und
- 0,2 bis 0,5 g basisches Magnesiumcarbonat.

13. Zusammensetzung nach einem der Ansprüche 3 bis 12, **dadurch gekennzeichnet, dass** sie eine kolloidale wässrige Zusammensetzung ist.

14. Zusammensetzung nach einem der Ansprüche 3 bis 13 zur Verwendung in der Human- und Veterinärtherapie.

15. Zusammensetzung zur Verwendung nach Anspruch 14 zur Behandlung und/oder Prävention von Erkrankungen des oberen Gastrointestinaltrakts, ösophagealem Reflux, Ösophagitis, Gastritis, Dyspepsie und peptischem Ulkus sowie als Antazidum, zytoprotektives und entzündungshemmendes Mittel der gastroösophagealen Schleimhaut.

## Revendications

1. Combinaison comprenant:
- de l'alginate de magnésium ayant un rapport entre les résidus d'acide mannuronique et les résidus d'acide guluronique (M/G) supérieur à 1,0, de préférence compris entre 1,5 et 3,0 ;
- du sucralfate en gel humide ; et
- du carbonate de magnésium basique.

2. Combinaison selon la revendication 1, **caractérisée en ce que** les rapports pondéraux entre l'alginate de magnésium/sucralfate en poids sec/carbonate de magnésium basique sont de 1/0,5 à 1/0,2-0,5.

3. Composition pharmaceutique aqueuse orale comprenant la combinaison selon la revendication 1 ou 2, ainsi qu'un ou plusieurs supports et/ou excipients pharmaceutiquement acceptables.

4. Composition selon la revendication 3, **caractérisée en ce qu'**elle comprend également un sel carbonate d'un métal alcalin.

5. Composition selon la revendication 3 ou 4, **caractérisée en ce que** ledit alginate de magnésium a un rapport M/G supérieur ou égal à 1,5, de préférence compris entre 1,5 et 3.

6. Composition selon la revendication 5, **caractérisée en ce que** ledit rapport est de 2,5.

7. Composition selon l'une quelconque des revendications 3 à 6, **caractérisée en ce que** ledit sucralfate est du sucralfate en gel humide ayant une teneur en eau comprise entre 60 et 80 % p/p.

8. Composition selon l'une quelconque des revendications 3 à 7, **caractérisée en ce que** ledit sel carbonate d'un métal alcalin est le carbonate de sodium.

9. Composition selon l'une quelconque des revendications 3 à 8, **caractérisée en ce qu'**elle comprend:
- 2,5 à 7,0 % d'alginate de magnésium, de préférence 3,0 à 5,5 % ;
- 10,0 à 40 % de sucralfate en gel humide, de préférence 20,0 à 39,0 % ; et
- 1,0 à 7,0 % de carbonate de magnésium basique, de préférence 2,0 à 6,0 %, plus préférablement 3,0 à 5,0 % ; le pourcentage étant exprimé en poids du composant par rapport au volume total de la composition, avec de l'eau et un ou plusieurs excipients pharmaceutiquement actifs.

10. Composition selon la revendication 8, **caractérisée en ce que** le sel carbonate d'un métal alcalin, lorsqu'il est présent, remplace 1/5 à 1/3 en poids de la quantité basique de carbonate de magnésium dans la composition.

11. Composition selon l'une quelconque des revendications 3 à 10, **caractérisée en ce qu'**elle se présente sous forme monodose ou multidoses.

12. Composition selon la revendication 11, **caractérisée en ce qu'**elle se présente sous forme monodose et chaque unité de dosage individuelle comprend:
- 0,2 à 2 g de sucralfate, sous forme sèche, et non sous forme de sucralfate en gel humide, de préférence 0,5 à 1,0 g ;
- 0,2 à 2 g d'alginate de magnésium, de préférence environ 0,5 g ; et
- 0,2 à 0,5 g de carbonate de magnésium basique.

13. Composition selon l'une quelconque des revendications 3 à 12, **caractérisée en ce qu'**il s'agit d'une composition aqueuse colloïdale.

14. Composition selon l'une quelconque des revendications 3 à 13, destinée à être utilisée en thérapie humaine et vétérinaire.

15. Composition destinée à être utilisée selon la revendication 14, dans le traitement et/ou la prévention des troubles du tractus gastro-intestinal supérieur, du reflux œsophagien, de l'œsophagite, de la gastrite, de la dyspepsie et de l'ulcère peptique, et comme agent antiacide, cytoprotecteur et anti-inflammatoire de la muqueuse gastro-oesophagienne.
